(19) Europäisches Patentamt
European Patent Office
Office européen des brevets

(11) Publication number: 0 503 520 A1

# (12) EUROPEAN PATENT APPLICATION

(21) Application number: 92103956.6

(22) Date of filing: 07.03.92

(51) Int. Cl.[5]: C07D 493/08, C07H 19/01, C12P 17/18, C12P 19/02, A61K 31/34, A61K 31/71, //(C07D493/08,319:00,307:00)

(30) Priority: 12.03.91 GB 9105152

(43) Date of publication of application: 16.09.92 Bulletin 92/38

(84) Designated Contracting States: AT BE CH DE DK ES FR GB GR IT LI LU MC NL PT SE

(71) Applicant: GLAXO GROUP LIMITED
Glaxo House, Berkeley Avenue
Greenford, Middlesex UB6 0NN(GB)

(72) Inventor: Rudd, Brian Arthur Michael
Glaxo Group Research Ltd. Berkeley Avenue
Greenford, Middlesex, UB6 0HE(GB)
Inventor: Noble, David
Glaxo Group Research Ltd. Berkeley Avenue
Greenford, Middlesex, UB6 0HE(GB)
Inventor: Hale, Richard Stephen
Glaxo Group Research Ltd. Berkeley Avenue
Greenford, Middlesex, UB6 0HE(GB)
Inventor: Sidebottom, Philip James
Glaxo Group Research Ltd. Berkeley Avenue
Greenford, Middlesex, UB6 0HE(GB)
Inventor: Hall, Richard Malcolm
Glaxo Group Research Ltd. Berkeley Avenue
Greenford, Middlesex, UB6 0HE(GB)

(74) Representative: Brewer, Christopher Laurence et al
Glaxo Holdings p.l.c. Glaxo House Berkeley Avenue
Greenford, Middlesex UB6 ONN(GB)

## (54) Cyclic ketal derivatives.

(57) Compounds are described of the formula

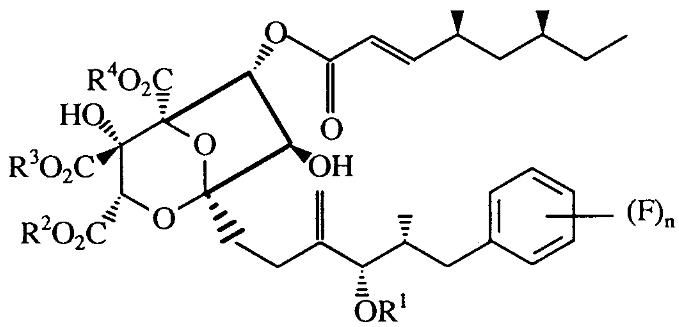

(I)

wherein $R^1$ represents a hydrogen atom or an acetyl group;
$R^2$, $R^3$ and $R^4$ may each independently represent a hydrogen atom or a methyl group; n represents an integer from 1 to 3; and the fluorine atom(s) present may be attached at the ortho, meta or para position of the benzene ring relative to the rest of the molecule; and salts thereof.

These compounds inhibit the enzyme squalene synthase and/or are intermediates for the preparation of compounds which inhibit the enzyme squalene synthase. Compounds of the invention may be formulated for

EP 0 503 520 A1

use in a variety of conditions where a lowering of the level of blood plasma cholesterol in animals would be beneficial and for use in combating fungal infections in animals.

This invention relates to novel compounds having hypocholesterolemic, hypolipidemic and/or antifungal activity, to processes for their preparation, to pharmaceutical compositions containing them and to their use in medicine, particularly in the treatment and/or prevention of atherosclerosis and associated cardiovascular diseases. The invention also relates to novel compounds which are useful as intermediates for the preparation of compounds having hypocholesterolemic, hypolipidemic and/or antifungal activity.

High levels of blood cholesterol and blood lipids are conditions which are implicated in the onset of vessel wall disease. Methods for effective reduction of plasma cholesterol levels are therefore of high interest. Cholesterol concentrations can be reduced, for example, by lowering the dietary intake of the sterol, by enhancing its metabolism and elimination or by decreasing its rate of biosynthesis. The most effective approaches to lowering physiological cholesterol levels are likely to include inhibition of cholesterol biosynthesis as a component since cholesterol synthesis is subject to feedback regulation, so that decreases in cholesterol levels tend to be compensated for by increased biosynthesis.

One rate-controlling step in the biosynthesis of cholesterol is the formation of mevalonic acid from 3-hydroxy-3-methylglutaryl coenzyme A (HMG CoA) and clinical successes have been achieved with the mevinic acid family of HMG CoA reductase inhibitors in the treatment of hypercholesterolemia. Mevalonic acid, however, is a common precursor of all isoprenyl derivatives, including in animals coenzyme Q, heme A and the dolichols.

The first biosynthetic step which leads exclusively to sterols, the condensation of two farnesyl pyrophosphates to give squalene, is a second site of regulation. The synthesis of squalene from farnesyl pyrophosphate involves an isolable intermediate, presqualene pyrophosphate, and the entire synthetic sequence is catalysed by squalene synthase (farnesyldiphosphate: farnesyldiphosphate farnesyltransferase, EC 2.5.1.21), a membrane-bound enzyme. Agents which act to inhibit the enzyme squalene synthase are therefore potential drugs for the regulation of cholesterogenesis. The use of such agents is attractive as non-steroidal pathways should be minimally affected.

The biosynthesis of ergosterol, the major sterol component of fungal cell membranes, is analogous to that of cholesterol in mammals and is thus mediated by the enzyme squalene synthase. Agents which act to inhibit the enzyme squalene synthase in fungal cells are therefore potential drugs for antifungal chemotherapy.

We have now found a group of novel compounds which act as inhibitors of the enzyme squalene synthase and/or intermediates for the preparation of compounds which act as inhibitors of the enzyme squalene synthase.

Thus, in a first aspect of the present invention, we provide compounds of the general formula (I)

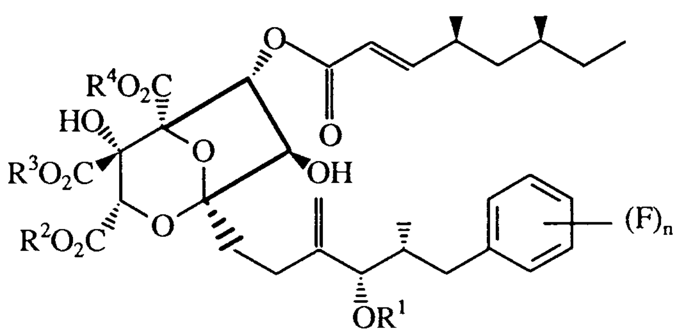

(I)

and salts thereof;
wherein $R^1$ represents a hydrogen atom or an acetyl group;
$R^2$, $R^3$ and $R^4$ may each independently represent a hydrogen atom or a methyl group; n represents an integer from 1 to 3; and the fluorine atom(s) present may be attached at the ortho, meta or para position of the benzene ring relative to the rest of the molecule.

Compounds of formula (I) in which $R^2$, $R^3$ and $R^4$ represent hydrogen atoms or physiologically acceptable cations are generally preferred.

A particular group of compounds of the invention are compounds of formula (I) in which $R^1$ represents an acetyl group, $R^2$, $R^3$ and $R^4$ each represent a hydrogen atom and n represents 1, and salts thereof.

Particularly preferred compounds of formula (I) are:
[1S-[1α(4R*,5S*),3α,4β,5α,6α(2E,4R*,6R*),7β]] 1-[4-acetoxy-6-(4-fluorophenyl)-5-methyl-3-methylenehexyl]-4,6,7-trihydroxy-2,8-dioxabicyclo[3.2.1]octane-3,4,5-tricarboxylic acid, 6-(4,6-dimethyl-2-octenoate);

[1S-[1α(4R*,5S*),3α,4β,5α,6α(2E,4R*,6R*),7β]] 1-[4-acetoxy-6-(3-fluorophenyl)-5-methyl-3-methylenehexyl]-4,6,7-trihydroxy-2,8-dioxabicyclo[3.2.1]octane-3,4,5-tricarboxylic acid, 6-(4,6-dimethyl-2-octenoate);
[1S-[1α(4R*,5S*),3α,4β,5α,6α(2E,4R*,6R*),7β]] 1-[4-acetoxy-6-(2-fluorophenyl)-5-methyl-3-methylenehexyl]-4,6,7-trihydroxy-2,8-dioxabicyclo[3.2.1]octane-3,4,5-tricarboxylic acid, 6-(4,6-dimethyl-2-octenoate);
and salts thereof.

Compounds of the present invention may form salts with inorganic and organic bases. Physiologically acceptable salts include inorganic base salts such as alkali metal salts (e.g. sodium and potassium salts including the trisodium, dipotassium and tripotassium salts), alkaline earth metal salts (e.g. calcium salts), ammonium salts (e.g. the diammonium salts) and amino acid salts (e.g. lysine and arginine salts including the tri-L-lysine salts). Suitable organic base salts include amine salts such as trialkylamine (e.g. triethylamine), dialkylamine (e.g. dicyclohexylamine), optionally substituted benzylamine (e.g. p-bromobenzylamine) and tris(hydroxymethyl)methylamine salts.

Compounds of the invention have been found to inhibit the enzyme squalene synthase and cholesterol biosynthesis and are therefore of use in medicine, particularly in a variety of conditions where a lowering of the level of blood plasma cholesterol in animals (especially humans) would be beneficial. Examples of such conditions include diseases associated with hypercholesterolemia and hyperlipoproteinemia, especially atherosclerosis and cardiovascular diseases (such as cardiac ischaemic diseases, cerebral ischaemic diseases and peripheral arterial disease).

Compounds of the invention which inhibit squalene synthase may also be of use in combating fungal infections in animals, including humans. For example, they may be useful in the treatment of systemic infections caused by, for example Candida (e.g. Candida albicans, Candida glabrata, Candida parapsilosis and Candida pseudotrop), Cryptococcus neoformans, Aspergillus Sp (e.g. Aspergillus flavus and Aspergillus fumigatus), Coccidioides (e.g. Coccidioides immitis), Paracoccidioides (e.g. Paracoccidioides brasiliensis), Histoplasma (e.g. Histoplasma capsulatum) or Blastomyces (e.g. Blastomyces dermatitidis). They may also be useful in treating topical infections caused by species of Trichophyton, Microsporum or Epidermophyton (e.g. Trichophyton mentographytes, Microsporum canis or Epidermophyton floccosum). They may also be of use in treating fungal diseases caused by Torulopsis glabrata and Pityrosporum ovale.

The in vitro evaluation of the anti-fungal activity of compounds of the invention can be performed by determining the minimum inhibitory concentration (MIC) which is the concentration of the test compound in a suitable medium at which growth of a particular microorganism fails to occur.

In view of their potential in antifungal therapy, compounds of the invention which inhibit squalene synthase may recommend themselves for the treatment of a variety of fungal infections in human beings and animals. Such infections include mycotic infections such as candidiasis and chronic mucocandidiasis (e.g. thrush and vaginal candidiasis) and skin infections caused by fungi, cutaneous and mucocutaneous candidiasis, dermatophytoses including ringworm and tinea infections, athletes foot, paronychia, pityriasis versicolor, erythrasma, intertrigo, fungal nappy rash, candida vulvitis, candida balanitis and otitis externa. They may also be useful as prophylactic agents to prevent systemic and topical fungal infections. Use as prophylactic agents may, for example, be appropriate as part of a selective gut decontamination regime in the prevention of infection in immunocompromised patients. Prevention of fungal overgrowth during antibiotic treatment may also be desirable in some disease syndromes or iatrogenic states.

The ability of compounds of the invention to inhibit the enzyme squalene synthase in mammals and fungi may be demonstrated in vitro using [2-$^{14}$C]farnesylpyrophosphate as a substrate under assay conditions similar to those described by S. A. Biller et al. in J. Medicinal Chemistry 31(10), 1869-1871 (1988); [$^{14}$C]squalene is separated from unreacted substrate on thin layer chromatography plates and determined by liquid scintillation counting. The ability of compounds of the invention to inhibit cholesterol biosynthesis may be demonstrated by measuring inhibition from [$^{14}$C]-acetate in liver slices from male Wistar rats using a method similar to that described by Y. Tsujita et al. in Biochem. Biophys. Acta, Volume 877, 50-60 (1986) and modified to include measurement of cholesterol by high performance liquid chromatography (h.p.l.c.).

While it is possible that, for use in therapy, compounds of the invention which inhibit squalene synthase may be administered as the raw chemical, it is preferable to present the active ingredient as a pharmaceutical formulation. The invention thus further provides a pharmaceutical formulation comprising a compound of the invention together with one or more pharmaceutically acceptable carriers thereof and, optionally, other therapeutic and/or prophylactic ingredients. The carrier(s) must be 'acceptable' in the sense of being compatible with the other ingredients of the formulation and not deleterious to the recipient thereof.

The compositions of the invention include those in a form especially formulated for oral, buccal, parenteral, implant, rectal, topical, ophthalmic or genito-urinary administration or in a form suitable for administration by inhalation or insufflation.

Tablets and capsules for oral administration may contain conventional excipients such as binding agents, for example, syrup, acacia, gelatin, sorbitol, tragacanth, mucilage of starch or polyvinylpyrrolidone; fillers, for example, lactose, sugar, microcrystalline cellulose, maize-starch, calcium phosphate or sorbitol; lubricants, for example, magnesium stearate, stearic acid, talc, polyethylene glycol or silica; disintegrants, for example, potato starch or sodium starch glycollate; or wetting agents such as sodium lauryl sulphate. The tablets may be coated according to methods well known in the art. Oral liquid preparations may be in the form of, for example, aqueous or oily suspensions, solutions, emulsions, syrups or elixirs, or may be presented as a dry product for constitution with water or other suitable vehicle before use. Such liquid preparations may contain conventional additives such as suspending agents, for example, sorbitol syrup, methyl cellulose, glucose/sugar syrup, gelatin, hydroxyethylcellulose, carboxymethyl cellulose, aluminium stearate gel or hydrogenated edible fats; emulsifying agents, for example, lecithin, sorbitan mono-oleate or acacia; non-aqueous vehicles (which may include edible oils), for example, almond oil, fractionated coconut oil, oily esters, propylene glycol or ethyl alcohol; and preservatives, for example, methyl or propyl p-hydroxybenzoates or sorbic acid. The compositions may also be formulated as suppositories, e.g. containing conventional suppository bases such as cocoa butter or other glycerides.

For buccal administration the composition may take the form of tablets or lozenges formulated in conventional manner.

The composition according to the invention may be formulated for parenteral administration by injection or continuous infusion. Formulations for injection may be presented in unit dose form in ampoules, or in multi-dose containers with an added preservative. The compositions may take such forms as suspensions, solutions, or emulsions in oily or aqueous vehicles, and may contain formulatory agents such as suspending, stabilising and/or dispersing agents. Alternatively the active ingredient may be in powder form for constitution with a suitable vehicle, e.g. sterile, pyrogen-free water, before use.

For administration by inhalation the compositions according to the invention are conveniently delivered in the form of an aerosol spray presentation from pressurised packs with the use of a suitable propellant, e.g. dichlorodifluoromethane, trichlorofluoromethane, dichlorotetrafluoroethane, carbon dioxide or other suitable gas, or from a nebuliser. In the case of a pressurised aerosol the dosage unit may be determined by providing a valve to deliver a metered amount.

Alternatively, for administration by inhalation the compositions according to the invention may take the form of a dry powder composition, for example, a powder mix of the compound and a suitable powder base such as lactose or starch. The powder composition may be presented in unit dosage form in, for example, capsules or cartridges of e.g. gelatin, or blister packs from which the powder may be administered with the aid of an inhaler or insufflator.

The compositions may take the form of a suppository, e.g. containing a conventional suppository base, or a pessary, e.g. containing a conventional pessary base.

The compositions may also be formulated for topical administration in the form of ointments, creams, gels, lotions, shampoos, powders (including spray powders), pessaries, tampons, sprays, dips, aerosols, drops (e.g. eye, ear or nose drops) or pour-ons. Ointments and creams may, for example, be formulated with an aqueous or oily base with the addition of suitable thickening and/or gelling agents. Ointments for administration to the eye may be manufactured in a sterile manner using sterilised components. Pour-ons may, for example, be formulated for veterinary use in oils containing organic solvents, optionally with formulatory agents, e.g. stabilising and solubilising agents. Pessaries and tampons for vaginal insertion may be formulated using conventional techniques and, where appropriate, may contain an effervescent vehicle. Such compositions may also contain other active ingredients such as corticosteroids, antibiotics or antiparasitics as appropriate.

Liquid preparations for intranasal delivery may take the form of solutions or suspensions and may contain conventional excipients such as tonicity adjusting agents, for example, sodium chloride, dextrose or mannitol; preservatives, for example benzalkonium chloride, thiomersal, phenylethyl alcohol; and other formulating agents such as suspending, buffering, stabilising and/or dispersing agents.

Transdermal administration may be affected by the design of a suitable system which promotes adsorption of the active compound through the skin and would typically consist of a base formulation enclosed within an adhesive stick-on patch comprising backing films, membranes and release liners.

The composition according to the invention may also be formulated as a depot preparation. Such long acting formulations may be administered by implantation (for example subcutaneously or intramuscularly) or by intramuscular injection. Thus, for example, a compound of the invention may be formulated with suitable polymeric or hydrophobic materials (for example as an emulsion in an acceptable oil) or ion exchange resins, or as sparingly soluble derivatives, for example, as a sparingly soluble salt.

When the compositions comprise dosage units, each unit will preferably contain 0.001mg to 1000mg,

advantageously 0.01mg to 400mg, of active ingredient where a compound of the invention is to be administered orally. The daily dosage as employed for adult human treatment will preferably range from 0.001mg to 5000mg of active ingredient, most preferably from 0.01mg to 2000mg which may be administered in 1 to 4 daily doses, for example, depending on the route of administration and on the condition of the patient and the disease to be treated.

The compound may be administered by intravenous infusion using, for example, up to 50mg/kg/day of the active ingredient. The duration of treatment will be dictated by the rate of response rather than by arbitrary numbers of days.

Compounds of the invention may also be used in combination with other therapeutic agents, and the invention thus provides, in a further aspect, a combination comprising a compound of the invention together with another therapeutically active agent, such as an inhibitor of 3-hydroxy-3-methylglutaryl coenzyme A (HMG CoA) reductase or another agent which reduces serum cholesterol and/or inhibits cholesterol biosynthesis, for example a bile acid sequestrant or an antihyperlipoproteinemic or antihyperlipemic agent such as probucol, gemfibrozil, clofibrate, dextrothyroxine or its sodium salt, colestipol or its hydrochloride salt, cholestyramine, nicotinic acid, neomycin, p-aminosalicylic acid, aspirin, DEAE-Sephadex, a poly-(diallylmethylamine) derivative, an ionene or poly(diallyldimethylammonium) chloride.

The combinations referred to above may conveniently be presented for use in the form of a pharmaceutical formulation and thus pharmaceutical formulations comprising a combination as defined above together with a pharmaceutically acceptable carrier thereof comprise a further aspect of the invention. The individual components of such combinations may be administered either sequentially or simultaneously in separate or combined pharmaceutical formulations.

When a compound of the invention is used in combination with a second therapeutic agent against the same condition the dose of each compound may differ from that when the compound is used alone. Appropriate doses will be readily appreciated by those skilled in the art.

According to another aspect of the present invention, we provide a compound of the invention or a pharmaceutical composition comprising a compound of the invention as defined above for use in therapy, particularly for the treatment of conditions where a lowering of the level of blood plasma cholesterol in animals (especially humans) would be beneficial, or for the treatment of fungal infections in animals (especially humans).

In a particular aspect of the present invention, we provide a compound of the invention or a pharmaceutical composition comprising a compound of the invention as defined above for use in the treatment of diseases associated with hypercholesterolemia and/or hyperlipoproteinemia, especially atherosclerosis and cardiovascular diseases (such as cardiac ischaemic diseases, cerebral ischaemic diseases and peripheral arterial disease).

According to a further aspect of the present invention, we provide the use of a compound of the invention in the manufacture of a medicament for the treatment of diseases associated with hypercholesterolemia and/or hyperlipoproteinemia, especially atherosclerosis and cardiovascular diseases (such as cardiac ischaemic diseases, cerebral ischaemic diseases and peripheral arterial disease).

According to another aspect of the present invention, we provide the use of a compound of the invention in the manufacture of a medicament for the treatment of fungal infections in a human or non-human animal patient.

According to a further aspect of the present invention, we provide a method of treatment of the human or non-human animal body to combat diseases associated with hypercholesterolemia and/or hyperlipoproteinemia, especially atherosclerosis and cardiovascular diseases (such as cardiac ischaemic diseases, cerebral ischaemic diseases and peripheral arterial disease) or to combat fungal diseases, which method comprises administering to said body an effective amount of a compound of the invention.

It will be appreciated by those skilled in the art that references herein to treatment extend to prophylaxis as well as the treatment of established conditions or infections.

The compounds of the invention may be prepared by the process described below.

Thus, according to a further aspect of the present invention, we provide a process for the preparation of the compounds of the invention which comprises the step of cultivating a microorganism capable of producing one or more of the compounds of the invention in the presence of a fluorinated benzoic acid or a precursor thereof and thereafter isolating the desired compound from the culture and, if desired, deacetylating the said compound and/or esterifying the said compound to the corresponding methyl ester.

Microorganisms capable of producing a compound of the invention in the presence of a fluorinated benzoic acid or a precursor thereof may readily be identified by using a small scale test and analysing a test sample obtained from fermentation of the microcrganism using standard methodology.

In particular the microorganism to be conventionally used is a strain of microorganism deposited in the

permanent culture collection of the CAB International Mycological Institute, Ferry Road, Kew, Surrey, England. The strain was received by the Institute on 25th May 1989 and was subsequently given the accession no. IMI 332962 and a deposit date of 27th June 1989 (date of confirmation of viability). The deposited strain is identified herein by reference to the Institute accession no. IMI 332962. The characteristics thus far identified for IMI 332962 are given in Example 2 hereinafter.

Mutants of the IMI 332962 may arise spontaneously or may be produced by a variety of methods including those outlined in Techniques for the Development of Micro-organisms by H. I. Adler in 'Radiation and Radioisotopes for Industrial Microorganisms', Proceedings of the Symposium, Vienna 1973, p241, International Atomic Energy Authority. Such methods include ionising radiation, chemical methods e.g. treatment with N-methyl-N'-nitro-N-nitrosoguanidine (NTG), heat, genetic techniques, such as recombination and transformation, and selective techniques for spontaneous mutants.

The genetic material of IMI 332962 and mutants thereof that participates in the synthesis of one or more of the compounds of the invention may be obtained using conventional genetic engineering techniques including those outlined by Rambosek and Leach in "Recombinant DNA in filamentous fungi : progress and prospects", CRC Critical Reviews in Biotechnology 1987, volume 6, pages 357-393.

Such techniques may be used in a similar manner to that described previously for cloning antibiotic biosynthetic genes for $\beta$-lactam antibiotic biosynthesis in Acremonium chrysogenum (Sansom, S.M. et al., 1985, Nature, 318, p191-194). The genetic material so obtained may be used, for example, for strain improvement as described by Skatrud, P. L. et al, 1989, Bio/Technology 7, pages 477-485), for production of biosynthetic enzymes for in vitro applications, or for generating novel compounds by introduction of such material into organisms other than IMI 332962.

In a further aspect of the invention we provide the bridged cyclic ketal derivatives obtainable from the fermentation of IMI 332962 or a mutant thereof in the presence of a fluorinated benzoic acid or a precursor thereof, deacetylated derivatives and methyl esters thereof.

In a particular aspect of the invention we provide the compounds having squalene synthase inhibitory activity obtainable from the fermentation of IMI 332962 or a mutant thereof in the presence of a fluorinated benzoic acid or a precursor thereof, deacetylated derivatives and methyl esters thereof.

Compounds suitable as precursors to fluorinated benzoic acids for use according to the fermentation process of the present invention may readily be identified using a convenient small scale fermentation and examining the effect of adding different compounds by analytical high performance liquid chromatography of the test sample. Examples of suitable compounds acting as precursors to fluorinated benzoic acids for use according to the fermentation process of the present invention include fluorinated phenylalanine, fluorinated cinnamic acid and fluorinated benzaldehyde.

References herein to fluorinated benzoic acid and precursors thereof mean mono-, di- or tri-fluorinated benzoic acid and precursors thereof as appropriate.

The production of compounds of the invention by fermentation of a suitable organism may be effected by conventional means i.e. by culturing the organism in the presence of assimilable sources of carbon, nitrogen and mineral salts and adding the fluorinated benzoic acid or a precursor thereof at any suitable time during the cultivation of the microorganism.

Assimilable sources of carbon, nitrogen and minerals may be provided by either simple or complex nutrients. Sources of carbon will generally include glucose, maltose, starch, glycerol, molasses, dextrin, lactose, sucrose, fructose, galactose, myo-inositol, D-mannitol, soya bean oil, carboxylic acids, amino acids, glycerides, alcohols, alkanes and vegetable oils. Sources of carbon will generally comprise from 0.5 to 10% by weight of the fermentation medium. Fructose, glucose and sucrose represent preferred sources of carbon.

Sources of nitrogen will generally include soya bean meal, corn steep liquors, distillers solubles, yeast extracts, cottonseed meal, peptones, ground nut meal, malt extract, molasses, casein, amino acid mixtures, ammonia (gas or solution), ammonium salts or nitrates. Urea and other amides may also be used. Sources of nitrogen will generally comprise from 0.1 to 10% by weight of the fermentation medium.

Nutrient mineral salts which may be incorporated into the culture medium include the generally used salts capable of yielding sodium potassium, ammonium, iron, magnesium, zinc, nickel, cobalt, manganese, vanadium, chromium, calcium, copper, molybdenum, boron, phosphate, sulphate, chloride and carbonate ions.

Cultivation of the organism will generally be effected at a temperature of from 20 to 40°C preferably from 20 to 35°C, especially around 25 to 28°C, and will desirably take place with aeration and agitation e.g. by shaking or stirring. The medium may initially be inoculated with a small quantity of mycelium and/or spores. The vegetative inoculum obtained may be transferred to the fermentation medium, or to one or more seed stages where further growth takes place before transfer to the principal fermentation medium.

The fluorinated benzoic acid or a precursor thereof may conveniently be added during the principle fermentation, e.g. about three days after the start of the principle fermentation, or fed into the fermentation medium as required. The quantity of fluorinated benzoic acid or a precursor thereof required will vary depending on the nature of the culture medium and the microorganism used and can generally be empirically determined to meet the requirements of each cultivation. The fermentation will generally be carried out in the pH range 3.5 to 9.5, preferably 4.5 to 7.5. It may be necessary to add a base or an acid to the fermentation medium to keep the pH within the desired range. Suitable bases which may be added include alkali metal hydroxides such as aqueous sodium hydroxide or potassium hydroxide. Suitable acids include mineral acids such as hydrochloric, sulphuric or phosphoric acid.

The fermentation may be carried out for an overall period of 4-30 days, preferably about 7-18 days. An antifoam may be present to control excessive foaming and added at intervals as required. Carbon and/or nitrogen sources may also be fed into the fermentation medium as required.

The compounds of the invention are present in the fermentation liquor, which may conveniently be separated by filtration or centrifugation. The liquor may be optionally thereafter treated with an acid such as sulphuric acid in the presence of an organic solvent until the pH is below pH 6 (e.g. about pH 3).

Compounds of the invention may be separated from the fermentation liquor by conventional isolation and separation techniques. It will be appreciated that the choice of isolation techniques may be varied widely.

Compounds of the invention may be isolated and purified by a variety of fractionation techniques, for example adsorption-elution, precipitation, fractional crystallisation, solvent extraction and liquid-liquid partition which may be combined in various ways.

Adsorption onto a solid support followed by elution has been found to be suitable for isolating and purifying compounds of the invention.

Compounds of the invention may be extracted with an appropriate organic solvent such as a ketone (e.g. acetone, methyl ethyl ketone or methyl isobutyl ketone), a halogenated hydrocarbon, an alcohol, a diol (e.g. propane-1,2-diol or butane-1,3-diol) or an ester (e.g. methyl acetate or ethyl acetate). Generally, more than one extraction may be desirable to achieve optimum recovery. The water-immiscible solvent extracts may themselves be extracted with basic aqueous solutions, and after acidification of these basic solutions the desired compounds may be reextracted into a water-immiscible organic phase. The solvent may then be removed from the organic extracts (e.g. by evaporation) to yield a material containing the desired compounds.

Chromatography (including high performance liquid chromatography) may be effected on a suitable support such as silica; a non-functional macroreticular adsorption resin, for example cross-linked styrene divinyl benzene polymer resins such as Amberlite XAD-2, XAD-4, XAD-16 or XAD-1180 resins (Rohm & Haas Ltd) or Kastell S112 (Montedison); a substituted styrene-divinyl benzene polymer, for example a halogenated (e.g. brominated) styrene-divinyl benzene polymer such as Diaion SP207 (Mitsubishi); an anion exchanger (e.g. IRA-35 or IRA-68) an organic solvent-compatible cross-linked dextran such as Sephadex LH20 (Pharmacia UK Ltd), or on reverse phase supports such as hydrocarbon linked silica e.g. $C_{18}$-linked silica. An alternative chromatographic means for the purification/separation of compounds of the invention is countercurrent chromatography using a coil extracter such as a multi-layer coil extracter.

Compounds of the invention may also be isolated and purified by the use of a liquid anion exchanger such as LA 2.

When IRA-68 or, particularly, IRA-35 is used as the solid adsorbant the extracts may be loaded directly at about pH3 or at about pH8 following filtration of solid impurities.

Suitable solvents/eluants for the chromatographic purification/ separation of compounds of the invention will, of course, depend on the nature of the column type and support. When using countercurrent chromatography a solvent system comprising ethyl acetate, hexane, methanol and an aqueous acid (e.g. aqueous sulphuric acid) may be particularly suitable. When using an anion exchanger such as IRA-35 the resin may conveniently be washed with aqueous acetone followed by elution with sulphuric acid in aqueous acetone.

The presence of compounds of the invention during the extraction/isolation procedures may be monitored by conventional techniques such as h.p.l.c. or UV spectroscopy or by utilising the properties of the compounds.

Where a compound of the invention is obtained in the form of a solution in an organic solvent, for example after purification by chromatography, the solvent may be removed by conventional procedures, e.g. by evaporation, to yield the required compound. If desired, the compound may be further purified by the aforementioned chromatographic techniques.

Deacetylation of compounds of the invention may be effected by acid or base catalysed hydrolysis.

Suitable bases include hydroxides such as sodium hydroxide and potassium hydroxide and alkoxides (e.g. methoxides). The base catalysed hydrolysis may take place in water optionally in the presence of an organic co-solvent such as an ether (e.g. tetrahydrofuran) or an alcohol (e.g. methanol) at a temperature in the range of $0^0$ to $100^0$C, preferably at elevated temperature. When an alkoxide is used as the base the reaction may conveniently be effected in an alcohol solvent (e.g. methanol) at a temperature in the range of $0^0$ to $100^0$C. Suitable acids include mineral acids (e.g. hydrochloric or sulphuric acid) and organic acids (e.g. p-toluenesulphonic acid). The acid catalysed hydrolysis may be carried out in water optionally in the presence of an organic co-solvent such as an ether (e.g. dioxan or tetrahydrofuran) or a ketone (e.g. acetone) at a temperature in the range of $0^0$ to $100^0$C, preferably at room temperature.

Esterification of compounds of the invention to the corresponding methyl esters may conveniently be effected by treatment with a methylating agent such as a methyl halide (e.g. methyl iodide) or dimethyl sulphate in a suitable organic solvent such as an amide (e.g. dimethylacetamide or preferably dimethylformamide) in the presence of a base such as a bicarbonate (e.g. sodium bicarbonate). The reaction may conveniently be carried out at a temperature ranging from $0^0$ to $100^0$C, preferably $20^0$ to $30^0$C. Alternatively, the esterification may be effected by treatment with an ethereal solution of diazomethane in a suitable solvent such as methanol. The esterification may also be effected by treatment with methanol in the presence of a suitable acid such as a mineral acid (e.g. hydrochloric acid) at about room temperature.

Conversion of one methyl ester of the invention to a different methyl ester of the invention may be carried out by appropriate esterification/deesterification steps. The deesterification may be effected under standard conditions, for example by base hydrolysis.

It is to be understood that the deacetylation and esterification processes may be combined as sequential or simultaneous reaction steps as appropriate.

Salts of compounds of the invention may be conveniently formed by treating a compound of the invention with an appropriate salt or base. Thus, for example, salts may conveniently be prepared by treating a compound of formula (I) with a salt or a base selected from sodium or potassium hydroxide, hydrogen carbonate, carbonate or acetate (e.g. potassium hydroxide, potassium hydrogen carbonate, sodium hydrogen carbonate or potassium acetate), ammonium acetate, calcium acetate and L-lysine as appropriate. The salt may, for example, be prepared by adding the appropriate salt or base (if necessary as an aqueous solution) to a solution or suspension of the compound of formula (I) in a suitable solvent such as water and/or a cosolvent such as an alcohol (e.g. methanol), a nitrile (e.g. acetonitrile) or a ketone (e.g. acetone) at temperatures of for example $0^0$C to $80^0$C and conveniently at about room temperature.

Salt formation may, if appropriate, be preceded by liberation of the compound of the invention from an aqueous-insoluble salt (e.g. a calcium salt) thereof by treating the aqueous-insoluble salt with a dilute aqueous mineral acid (e.g. hydrochloric or sulphuric acid), followed by batch adsorption onto reverse phase silica and elution with an appropriate organic solvent.

The isolated compounds of the invention and salts thereof may be further purified by filtering a solution (e.g. an acetone solution) of the compound under sterile conditions and recrystallising the compound from the filtrate.

The following examples are provided by way of illustrating the invention and are not intended to limit the invention in any way.

Example 1

IMI 332962 was grown on agar plates of the following composition:

| | |
|---|---|
| Malt extract (Oxoid L39) | 30g |
| Mycological peptone (Oxoid L40) | 5g |
| Yeast extract (Oxoid L21) | 0.5g |
| Agar (Oxoid No 3) | 20g |
| Distilled water to 1 litre | |

The pH of the medium before autoclaving was in the range of 5.3-5.5. The inoculated plates were incubated at $28^0$C for 14 days. Several 6mm diameter plugs of agar covered with fungal mycelium were cut from the growing edge of the culture and two plugs were transferred into each of several cryotubes containing 1.6ml of sterile distilled water. The tubes were capped and stored at $-20^0$ until required.

0.5ml of IMI 332962 seed stock was used to inoculate 50ml of seed medium (A) contained in a 250ml Erlenmeyer flask:

| Seed medium (A) : | |
|---|---|
| Peptone (Oxoid L34) | 10g |
| Malt extract (Oxoid L39) | 21g |
| Glycerol | 40g |
| Junlon 110 (Honeywill & Stein Ltd., Wallington, Surrey) | 1g |
| Distilled water to 1 litre | |

The pH of the medium was adjusted to 6.3-6.5 with aqueous sodium hydroxide before autoclaving

The flask of inoculated seed medium was incubated at 25$^0$C on a shaker platform, which rotated at 250rpm with a 50mm diameter orbital motion, for 3 days. The culture was then passed through a 50ml syringe and 1ml was used to inoculate a 50ml test flask containing fermentation medium (B)

| Fermentation medium (B) : | |
|---|---|
| fructose | 50g/L |
| soya bean oil | 30g/L |
| cotton seed flour | 20g/L (Sigma) |

The flask was incubated as above for three days at which time a fluorinated benzoic acid or precursor was added (solution in water 6.25 - 12.5mg/ml, filter sterilised or directly as a liquid as appropriate) to a final concentration of 250mg/L. The flask was incubated as before for a further 4 or 5 days. 1ml of whole broth was then mixed with an equal volume of extraction mixture (acetonitrile containing concentrated sulphuric acid 5ml/L) and left to stand for 30 minutes. The tube was then centrifuged for 10 minutes and the supernatant was decanted into a fresh tube. The centrifugation was then repeated to provide a clarified supernatant.

Product formation was determined by analysing the supernatant material by gradient high performance liquid chromatography (Spherisorb 5 $\mu$M C6 column; 15cm x 4.6mm i.d.) using acidified aqueous acetonitrile as eluant with detection at 210nm. Those samples which produced novel products were scaled up as described hereinafter.

(a) [1S-[1$\alpha$(4R*,5S*),3$\alpha$,4$\beta$,5$\alpha$,6$\alpha$(2E,4R*,6R*),7$\beta$]] 1-[4-Acetoxy-6-(4-fluorophenyl)-5-methyl-3-methylenehexyll-4,6,7-trihydroxy-2,8-dioxabicyclo[3.2.1]octane-3,4,5-tricarboxylic acid, 6-(4,6-dimethyl-2-octenoate)

The pooled contents of 20 shake flasks (ca. 800ml) prepared as described above and fed with p-fluorobenzoic acid were adjusted to pH 10.4 with 0.880 ammonia solution, centrifuged (5000g) for 30 minutes and the cells and supernatant separated. The cells were suspended in water (250ml) and the suspension was adjusted to pH 10.4 with 0.880 ammonia solution, then centrifuged as before. The supernatants from each centrifugation were combined, adjusted to pH 8.2 and loaded onto a column of Amberlite XAD16(22 x 2.6cm i.d.) at 1.5 bed volumes per hour. The column was washed with water (500ml), tetrasodium edetate (1% w/v, 500ml), water (500ml) and eluted with acetone/water (6:4). The eluate containing the required component (90ml to 150ml after commencement of elution) was diluted with an equal volume of water and then acidified with sulphuric acid (2.4ml) and pumped onto a column (25 x 2.1cm i.d.) of Spherisorb S5 ODS2. The column was washed with acetonitrile/0.095N sulphuric acid (1:3, 1L) and eluted with acetonitrile/0.13N sulphuric acid (45:55). The fraction eluting between 875ml to 1175ml was diluted with an equal volume of water and pumped back onto the cleaned, water-equilibrated column. The column was washed with water until free of acid and then eluted with acetonitrile/water (9:1). The eluate was evaporated to yield an oily residue (3ml) to which was added acetonitrile (2.45ml) and sulphuric acid (11$\mu$L). The solution was chromatographed in two portions on a column of Spherisorb S5 ODS2 (25 x 2.1cm i.d.), in acetonitrile/0.13N sulphuric acid (45:55), at 25ml/min with detection at 210nm. The component eluting from the column between 36 and 40 minutes in both runs was collected and the combined solutions were diluted with an equal volume of water and pumped back onto the cleaned, water-equilibrated column. The column was washed with water (1.5L) and eluted with acetonitrile/water (9:1). The eluate was evaporated in vacuo and the aqueous suspension was freeze-dried to give the title compound as a white powder (1.2mg); -FAB mass spectrometry $[M-H]^-$707; molecular weight ca. 708; molecular formula $C_{35}H_{45}FO_{14}$; 500MHz proton nmr spectrum in deutero-methanol includes signals [$\delta$ values with multiplic-

ities, coupling constants (Hz) and integration values in parentheses] centred at about : 0.83 to 0.90 (m,9H), 1.03 (d,7,3H), 2.10 (s,3H), 2.25 (m,1H), 2.36 (m,1H), 2.67 (dd,14,6,1H), 4.02 (d,2,1H), 4.96 (s,1H), 5.02 (s,1H), 5.08 (d,5,1H), 5.27 (s,1H), 5.81 (dd,16,1,1H), 6.33 (d,2,1H), 6.85 (dd,16,8,1H), 6.99 (t,8,2H), 7.21 (dd,8,5,2H).

(b) [1S-[1α(4R*,5S*),3α,4β,5α,6α(2E,4R*,6R*),7β]] 1-[4-Acetoxy-6-(3-fluorophenyl)-5-methyl-3-methylenehexyl]-4,6,7-trihydroxy-2,8-dioxabicyclo[3.2.1]octane-3,4,5-tricarboxylic acid, 6-(4,6-dimethyl-2-octenoate)

The pooled contents of 40 shake flasks (ca. 1800ml) prepared as described above and fed with m-fluorobenzoic acid were adjusted to pH 10.4 with 0.880 ammonia solution, centrifuged (5000g) for 30 minutes and the supernatant separated. The cells were suspended in water (1L) and the suspension was adjusted to pH 10.4 with 0.880 ammonia solution, then centrifuged as before. The supernatants from each centrifugation were combined, adjusted to pH 8.2 with sulphuric acid and loaded onto a column of Amberlite XAD16 (23 x 3.5cm i.d.) at about 1 bed volume/hour. The column was washed with water (500ml), tetrasodium edetate (1% w/v; 500ml), water (1L) and eluted with acetone/water (6:4). The eluate that contained the required component (from 250ml after commencement of elution to ca. 500ml) was diluted with water (1:1) and acidified with sulphuric acid, then pumped onto a column of Spherisorb S5 ODS2 (25 x 2.1cm i.d.). The column was washed with acetonitrile/0.095N sulphuric acid (1:3, 300ml), then eluted with acetonitrile/0.13N sulphuric acid (45:55). The fraction eluting between 225ml and 800ml was diluted with an equal volume of water and pumped back onto the cleaned, water-equilibrated column. The column was washed with water until free of acid and then eluted with acetonitrile/water (9:1). The eluate was evaporated to give an oily residue (3ml) to which was added acetonitrile (2.45ml) and sulphuric acid (0.011ml). The solution was chromatographed in 10 portions on a column of Spherisorb S5 ODS2 (25 x 2.1cm i.d.) in acetonitrile/0.13N sulphuric acid (45:55) at 25ml/min, with detection at 210nm. The fraction eluting between 53 and 57 minutes in each run was collected and the combined solutions were diluted with an equal volume of water and pumped back onto the cleaned, water-equilibrated column. The column was washed with water (2L) and eluted with acetonitrile/water (9:1). The eluate was evaporated in vacuo and the aqueous suspension was freeze-dried to give the title compound as a white powder (29.4mg); -FAB mass spectrometry $[M-H]^-$ 707; molecular weight ca. 708; molecular formula $C_{35}H_{45}FO_{14}$; 500MHz proton nmr spectrum in deutero-methanol includes signals [δ values with multiplicities, coupling constants (Hz) and integration values in parentheses] centred at about: 0.83 to 0.90 (m,9H), 1.03 (d,7,3H), 2.11 (s,3H), 2.25 (m,1H), 2.34 (m,1H), 2.72 (dd,13,6,1H), 4.06 (d,2,1H), 4.98 (s,1H), 5.03 (s,1H), 5.10 (d,5,1H), 5.27 (s,1H), 5.80 (dd,16,1,1H), 6.32 (d,2,1H), 6.84 (dd,16,8,1H), 6.88 (td,9,2,1H), 6.94 (dt,10,2,1H), 7.03 (d,8,1H), 7.28 (dt,6,8,1H).

(c) [1S-[1α(4R*,5S*),3α,4β,5α,6α(2E,4R*,6R*),7β]] 1-[4-Acetoxy-6-(2-fluorophenyl)-5-methyl-3-methylenehexyl]-4,6,7-trihydroxy-2,8-dioxabicyclo[3.2.1]octane-3,4,5-tricarboxylic acid, 6-(4,6-dimethyl-2-octenoate)

The pooled contents of 99 shake flasks (ca. 4.5L) prepared as described above and fed with o-fluorobenzaldehyde were adjusted to pH 2 with concentrated sulphuric acid, centrifuged (5000g) for 30 minutes and the supernatant separated. The cells were suspended in acetonitrile/water (1:1) containing 0.5ml/L sulphuric acid (3L), then the acetonitrile removed by evaporation in vacuo to give an aqueous solution. This solution was loaded onto a column of Amberlite XAD16 (26 x 3.5cm i.d.) at about 1 bed volume/hour. The column was washed with water (500ml), tetrasodium edetate (1% w/v; 500ml), water (500ml) then eluted with acetone/water (6:4). The eluate that contained the required component (from ca. 300ml after commencement of elution to 800ml) was diluted with an equal volume of water and acidified with concentrated sulphuric acid (2ml), then pumped onto a column of Spherisorb S5 ODS2 (25 x 2.1cm i.d.). The column was washed with acetonitrile/0.095N sulphuric acid (1:3, 1.4L) then eluted with acetonitrile/0.13N sulphuric acid (45:55). The fraction eluting between 550ml and 1350ml after commencement of elution was collected and diluted with an equal volume of water. This solution was pumped back onto the cleaned, water-equilibrated column. The column was washed with water (1.5L) then eluted with acetonitrile/water (9:1). The eluate was evaporated to yield a concentrated extract (5ml) to which was added acetonitrile (4.05ml) and concentrated sulphuric acid (0.018ml). The solution was chromatographed in seven portions on a column of Spherisorb S5 ODS2 (25 x 2.1cm i.d.) in acetonitrile/0.13N sulphuric acid (45:55) at 25ml/min with detection at 210nm. The component eluting between 42 and 50 mins was collected from each run and the combined solutions were diluted with an equal volume of water. This solution was pumped

back onto the cleaned, water-equilibrated column and the column was washed with water (1.75L) and eluted with acetonitrile/water (9:1). The eluate was evaporated in vacuo to give a concentrated extract (4.25ml). To this extract was added ethanol (5.75ml) and phosphoric acid (0.02ml) and the solution was centrifuged (12000g) for 5 minutes. The supernatant was chromatographed in 10 portions on a column of Spherisorb C6 5μM (25 x 2.1cm i.d.) with methanol/water (57.5:42.5) containing phosphoric acid (75μL/L, pH 3) at 22.5ml/min with detection at 210nm. The component from each run eluting between 90 and 95 mins was collected. The combined solutions were diluted with an equal volume of water and pumped back onto the cleaned, water-equilibrated column. The column was washed with water (1.5L) and eluted with acetonitrile/water (9:1). The eluate was evaporated in vacuo to give about 1ml concentrated solution. To this solution was added methanol (4ml) and phosphoric acid (0.01ml) and this solution was filtered. The filtrate was chromatographed on a column of Spherisorb C6 5μM (25 x 2.1cm i.d.) with methanol/water (57.5:42.5) containing phosphoric acid (75μL/L, pH3) at 22.5 ml/min with detection at 210nm. The component eluting between 80 and 91 mins was collected and diluted with an equal volume of water and pumped back onto the cleaned, water-equilibrated column. The column was washed with water (2L) and eluted with acetonitrile/water (9:1). The eluate was evaporated in vacuo to remove acetonitrile and the aqueous suspension was freeze dried to yield the title compound as a white powder (2mg); -FAB mass spectrometry $[M-H]^-$707; molecular weight ca. 708; molecular formula $C_{35}H_{45}FO_{14}$; 500MHz proton nmr spectrum in deutero-methanol includes signals [δ values with multiplicities, coupling constants (Hz) and integration values in parentheses] centred at about : 0.83 to 0.90 (m,9H), 1.03 (d,7,3H), 2.10 (s,3H), 2.72 (dd,13,6,1H), 4.00 (s,1H), 4.96 (s,1H), 5.02 (s,1N), 5.07 (d,5,1H), 5.24 (s,1H), 5.83 (d,16,1H), 6.31 (s,1N), 6.84 (dd,16,8,1H), 7.00 (m,1H), 7.09 (dt,1,7,1H), 7.18 (m,1H), 7.28 (dt,1,7,1H).

The above fermentation procedure was repeated fed with o-fluorobenzoic acid, m-fluorobenzaldehyde, p-fluorobenzaldehyde, o-, m- and p-fluorocinnamic acid and o-, m- and p-fluorophenylalanine. Analysis of the broth (by high performance liquid chromatography/ mass spectrometry) from each experiment indicated the presence of the appropriate o-, m- or p-fluoro substituted compound of formula (I). The titre of desired product from each experiment is provided in the following table.

| COMPOUND | FEEDING AGENT | TITRE (mg/L) |
|---|---|---|
| Example 1(a) | p-fluorocinnamic acid<br>p-fluorobenzaldehyde<br>p-fluorophenylalanine | 16<br>5<br>13 |
| Example 1(b) | m-fluorocinnamic acid<br>m-fluorobenzaldehyde<br>m-fluorophenylalanine | 67<br>15<br>30 |
| Example 1(c) | o-fluorobenzoic acid<br>o-fluorocinnamic acid<br>o-fluorophenylalanine | 30<br>12<br>2 |

## Example 2

### Characteristics of IMI 332962

After 2-3 weeks growth at 25°C on oatmeal agar the colonies were smoke grey to mouse grey in colour (Rayner's Mycological Colour Chart, 1970; published by the Commonwealth Agricultural Bureaux) on both the surface and reverse of the colony.

Morphological observations of the strain grown at 25°C on oatmeal agar were made under an optical microscope. The fungus had no sexual reproductive stage but formed pycnidia, thereby placing it in the class Coelomycetes. The fungus produced rostrate pycnidia with loose hyphae and both aseptate and one-septate conidia. The conidia were approximately 5-9 x 1.5-3μM in dimensions (usually 7-9 x 1.502.5μM). Numerous multiseptate/multicellular, globose structures resembling chlamydospores or pycnidial initials were also observed. Distinct dictyochlamydospores were absent.

The isolate has been identified as a species of the genus *Phoma*, and the identity confirmed by the CAB International Mycological Institute.

### IN VITRO RESULTS

The ability of compounds of the invention to inhibit the enzyme squalene synthase was demonstrated using [2-$^{14}$C] farnesylpyrophosphate as substrate under assay conditions similar to those described by S. A. Biller et al in J Medicinal Chemistry 31(10), 1869-1871(1988). [$^{14}$C] Squalene was separated from unreacted substrate on thin layer chromatography plates and determined by liquid scintillation counting. Inhibition of squalene synthase was quantified by incubating rat liver homogenate with various concentrations of the test compound in the presence of [2-$^{14}$C] farnesylpyrophosphate. The concentration of compound giving 50% inhibition of [$^{14}$C] squalene production in a 30 minute assay was taken as the $IC_{50}$ value.

In this test the title compounds of Examples 1(a), 1(b) and 1(c) had $IC_{50}$ values of less than 100nM.

Pharmaceutical Examples

In the following examples the term 'Active Ingredient' refers to a compound of the present invention, for example a compound described in the Examples hereinabove.

Example 1 - Tablets

a)

| | |
|---|---|
| Active Ingredient | 5.0mg |
| Lactose | 95.0mg |
| Microcrystalline Cellulose | 90.0mg |
| Cross-linked Polyvinylpyrrolidone 8.0mg | |
| Magnesium Stearate | 2.0mg |
| Compression Weight | 200.0mg |

The active ingredient, microcrystalline cellulose, lactose and cross-linked polyvinylpyrrolidone are sieved through a 500 micron sieve and blended in a suitable mixer. The magnesium stearate is sieved though a 250 micron sieve and blended with the active blend. The blend is compressed into tablets using suitable punches.

b)

| | |
|---|---|
| Active Ingredient | 5.0mg |
| Lactose | 165.0mg |
| Pregelatinised Starch | 20.0mg |
| Cross-linked Polyvinylpyrrolidone 8.0mg | |
| Magnesium Stearate | 2.0mg |
| Compression weight | 200.0mg |

The active ingredient, lactose and pregelatinised starch are blended together and granulated with water. The wet mass is dried and milled. The magnesium stearate and cross-linked polyvinylpyrrolidone are screened through a 250 micron sieve and blended with the granule. The resultant blend is compressed using suitable tablet punches.

Example 2 - Capsules

a)

| | |
|---|---|
| Active Ingredient | 5.0mg |
| Pregelatinised Starch | 193.0mg |
| Magnesium Stearate | 2.0mg |
| Fill weight | 200.0mg |

The active ingredient and pregelatinised starch are screened through a 500 micron mesh sieve, blended together and lubricated with magnesium stearate (meshed through a 250 micron sieve). The blend is filled into hard gelatin capsules of a suitable size.

b)

| | |
|---|---|
| Active Ingredient | 5.0mg |
| Lactose | 177.0mg |
| Polyvinypyrrolidone | 8.0mg |
| Cross-linked Polyvinylpyrrolidone 8.0mg | |
| Magnesium Stearate | 2.0mg |
| Fill weight | 200.0mg |

The active ingredient and lactose are blended together and granulated with a solution of polyvinylpyrrolidone. The wet mass is dried and milled. The magnesium stearate and cross-linked polyvinylpyrrolidone are screened through a 250 micron sieve and blended with the granule. The resultant blend is filled into hard gelatin capsules of a suitable size.

Example 3 - Syrup

a)

| | |
|---|---|
| Active Ingredient | 5.0mg |
| Hydroxypropyl Methylcellulose 45.0mg | |
| Propyl Hydroxybenzoate | 1.5mg |
| Butyl Hydroxybenzoate | 0.75mg |
| Saccharin Sodium | 5.0mg |
| Sorbitol Solution | 1.0ml |
| Suitable Buffers | qs |
| Suitable Flavours | qs |
| Purified Water to | 10.0ml |

The hydroxypropyl methylcellulose is dispersed in a portion of hot purified water together with the hydroxybenzoates and the solution is allowed to cool to room temperature. The saccharin sodium, flavours and sorbitol solution are added to the bulk solution. The active ingredient is dissolved in a portion of the remaining water and added to the bulk solution. Suitable buffers may be added to control the pH in the region of maximum stability. The solution is made up to volume, filtered and filled into suitable containers.

Example 4- Intranasal Solution

a)

| Preserved solution | |
|---|---|
| | % w/w |
| Active Ingredient | 0.1 |
| Dextrose (Anhydrous) | 5.0 |
| Benzalkonium Chloride | 0.02 |
| Suitable buffers | qs |
| Purified Water to | 100 |

The active ingredient and dextrose are dissolved in a portion of the bulk solution. Suitable buffers may be added to control the pH in the region of maximum stability. The solution is made up to volume, filtered and filled into suitable containers.

Alternatively, the solution may be provided as a sterile unit dose presentation such that the preservatives are omitted from the formulation.

b)

| Unpreserved sterile solution | |
|---|---|
| | % w/w |
| Active Ingredient | 0.1 |
| Dextrose (Anhydrous) | 5.0 |
| Suitable Buffers | qs |
| Purified Water to | 100 |

## Claims

**1.** Compounds having the formula (I)

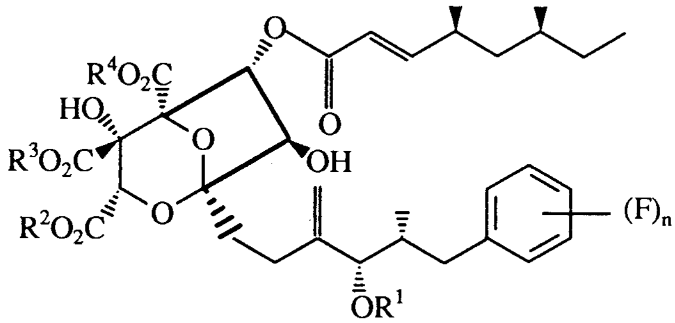

(I)

wherein $R^1$ represents a hydrogen atom or an acetyl group; $R^2$, $R^3$ and $R^4$ may each independently represent a hydrogen atom or a methyl group; n represents an integer from 1 to 3; and the fluorine atom(s) present may be attached at the ortho, meta or para position of the benzene ring relative to the rest of the molecule; and salts thereof.

**2.** Compounds according to Claim 1 in which $R^2$, $R^3$ and $R^4$ represent hydrogen atoms.

**3.** Compounds according to Claim 1 in which $R^1$ represents an acetyl group, $R^2$, $R^3$ and $R^4$ each represent a hydrogen atom and n represents 1, and salts thereof.

**4.** [1S-[1α(4R*,SS*),3α,4β,5α,6α(2E,4R*,6R*),7β]] 1-[4-Acetoxy-6-(4-fluorophenyl)-5-methyl-3-methylenehexyl]-4,6,7-trihydroxy-2,8-dioxabicyclo[3.2.1]octane-3,4,5-tricarboxylic acid, 6-(4,6-dimethyl-2-octenoate); and salts thereof.

**5.** [1S-[1α(4R*,5S*),3α,4β,5α,6α(2E,4R*,6R*),7β]] 1-[4-Acetoxy-6-(3-fluorophenyl)-5-methyl-3-methylenehexyl]-4,6,7-trihydroxy-2,8-dioxabicyclo[3.2.1]octane-3,4,5-tricarboxylic acid, 6-(4,6-dimethyl-2-octenoate): and salts thereof.

**6.** [1S-[1α(4R*,5S*),3α,4β,5α,6α(2E,4R*,6R*),7β]] 1-[4-Acetoxy-6-(2-fluorophenyl)-5-methyl-3-methylenehexyl]-4,6,7-trihydroxy-2,8-dioxabicyclo[3.2.1]octane-3,4,5-tricarboxylic acid, 6-(4,6-dimethyl-2-octenoate); and salts thereof.

**7.** A compound according to any preceding claim in substantially pure form.

**8.** A compound according to any preceding claim for use in therapy.

**9.** A compound according to any preceding claim for use in the treatment of conditions where a lowering of the level of blood plasma cholesterol in animals, including humans, would be beneficial.

**10.** A compound according to any of Claims 1 to 7 for use in the treatment of fungal infections in a human or non-human animal patient.

**11.** A method of treatment of the human or non-human animal body to combat diseases associated with hypercholesterolemia and/or hyperlipoproteinemia or to combat fungal diseases, which method comprises administering to said body an effective amount of a compound as claimed in any of Claims 1 to 7 which inhibits squalene synthase.

**12.** A pharmaceutical composition comprising a compound according to any of Claims 1 to 7 together with one or more carriers and/or excipients.

**13.** A pharmaceutical composition comprising an active amount of a compound as claimed in any of Claims 1 to 7 for use in the treatment of conditions where a lowering of the level of blood plasma cholesterol in animals, including humans, would be beneficial.

**14.** A pharmaceutical composition comprising an active amount of a compound as claimed in any of Claims 1 to 7 for use in the treatment of fungal infections in a human or non-human animal patient.

**15.** A pharmaceutical composition according to any one of Claims 12 to 14 in a form suitable for oral, buccal, topical, parenteral, implant, rectal, ophthalmic or genito-urinary administration or in a form suitable for administration by inhalation or insufflation.

**16.** A pharmaceutical composition according to any one of Claims 12 to 15 in unit dosage form.

**17.** Use of a compound according to any of Claims 1 to 7 in the manufacture of a medicament for the treatment of hypercholesterolemia and/or hyperlipoproteinemia in a human or non-human animal patient.

**18.** Use of a compound according to any of Claims 1 to 7 in the manufacture of a medicament for the treatment of fungal infections in a human or non-human animal patient.

**19.** A process for the preparation of a compound as claimed in any of Claims 1 to 7 which comprises the step of cultivating IMI 332962 or a mutant thereof in the presence of a fluorinated benzoic acid or a precursor thereof and thereafter isolating one or more such compounds from the culture, followed if desired by deacetylation and/or conversion to a methyl ester to provide the desired compound.

**20.** Bridged cyclic ketal derivatives obtainable by a process according to Claim 19.

**21.** Squalene synthase inhibitors whenever produced by a procedure including a process according to Claim 19.

**22.** Deacylated derivatives of compounds according to Claim 21 and methyl esters thereof.

**23.** Compounds according to any of Claims 1 to 7 substantially as herein described.

**24.** Compositions according to any one of Claims 12 to 16 substantially as herein described.

European Patent Office

# PARTIAL EUROPEAN SEARCH REPORT

which under Rule 45 of the European Patent Convention shall be considered, for the purposes of subsequent proceedings, as the European search report

Application Number

EP 92 10 3956

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| P,X | EP-A-0 450 812 (MERCK & CO.)<br>* Claims 1,6,8,10 *<br>--- | 1,17-19 | C 07 D 493/08<br>C 07 H 19/01<br>C 12 P 17/18<br>C 12 P 19/02<br>A 61 K 31/34<br>A 61 K 31/71 //<br>(C 07 D 493/08<br>C 07 D 319:00<br>C 07 D 307:00 ) |
| A | EP-A-0 259 087 (MERCK & CO.)<br>* Claims 1,9 *<br>----- | 1,13 | |

TECHNICAL FIELDS SEARCHED (Int. Cl.5)

C 07 D
C 07 H
C 12 P
A 61 K

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely :

Claims searched incompletely :

Claims not searched :

Reason for the limitation of the search:

Remark: Although claim 11 is directed to a method of treatment of (diagnostic method practised on) the human/animal body (Article 52(4) EPC) the search has been carried out and based on the alleged effects of the compound/composition.

| Place of search | Date of completion of the search | Examiner |
|---|---|---|
| THE HAGUE | 13-05-1992 | VOYIAZOGLOU D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0407)